Europäisches Patentamt

European Patent Office

Office européen des brevets

(19)

(11) Veröffentlichungsnummer: **0 080 436**
**B1**

(12) **EUROPÄISCHE PATENTSCHRIFT**

(45) Veröffentlichungstag der Patentschrift:
14.01.87

(21) Anmeldenummer: **82810500.7**

(22) Anmeldetag: **23.11.82**

(51) Int. Cl.⁴: **A 61 B 17/22**, A 61 M 25/00

(54) Vorrichtung zur Entfernung bzw. zur Aufweitung von Engstellen in Körperflüssigkeit führenden Gefässen.

(30) Priorität: 24.11.81 DE 3146459
29.09.82 DE 3235974

(43) Veröffentlichungstag der Anmeldung:
01.06.83 Patentblatt 83/22

(45) Bekanntmachung des Hinweises auf die Patenterteilung:
14.01.87 Patentblatt 87/3

(84) Benannte Vertragsstaaten:
AT BE CH DE FR GB IT LI LU NL SE

(56) Entgegenhaltungen:
DE-A-2 816 391
DE-A-2 834 956
DE-A-2 848 484
DE-A-2 933 266
DE-B-1 069 823
FR-A-2 350 849
GB-A-2 054 385
US-A-2 687 131

(73) Patentinhaber: Schneider Medintag AG,
Schärenmoosstrasse 115, CH- 8052 Zürich (CH)

(72) Erfinder: Weikl, Andreas, Klosbacher Weg 51 b,
D-8520 Erlangen (DE)
Erfinder: Merkel, Volkmar, Faust- von- Stromberg-
Strasse 1, D-8520 Erlangen (DE)

(74) Vertreter: Isler, Fritz, Dipl.- Ing.,
PATENTANWALTS- BUREAU ISLER AG Postfach
6940 Walchestrasse 23, CH- 8023 Zürich (CH)

EP 0 080 436 B1

**Beschreibung**

Die vorliegende Erfindung bezieht sich auf einen Behandlungskatheter zur Entfernung bzw. Aufweitung von Engstellen in Körperflüssigkeit führenden Gefäßen.

Aus der FR-A-2 350 849 ist bereits ein Angiographiekatheter mit zwei in axialer Richtung im Abstand voneinander angeordneten ausdehnbaren Ballons bekannt, von denen der eine Ballon im aufgeblasenen Zustand den Bereich der Behandlung rückseitig abdichtet, während der andere Ballon im aufgeblasenen Zustand diesen Bereich vorderseitig abdichtet, wobei der Ausdehnungsgrad beider Ballons von außen regulierbar ist und der Katheter durch eine Längstrennwand in zwei Kanäle unterteilt ist, von denen der eine Kanal das Druckmittel zum Aufblasen des einen Ballons und der andere Kanal das Druckmittel zum Aufblasen des anderen Ballons führt und im Bereich zwischen den beiden Ballons die Wandung des Katheters mit einer Öffnung versehen ist, die in den von den beiden Ballons abgedichteten Bereich mündet und mit einem der beiden Kanäle zur Führung des Druckmittels in Verbindung steht.

Bei einem aus der DE-B-1 069 823 bekannten Behandlungskatheter sind am gefäßseitigen Ende desselben wenigstens zwei ausdehnbare Ballons im Abstand hintereinander vorgesehen. Jeder Ballon ist mit einem von außen zugänglichen Kanal verbunden und kann individuell durch äußeren Druck aufgebläht werden. Nach Einführung des Behandlungskatheters in einen Harnleiter kann der Harnleiter durch die Aufblähung der Ballons gedehnt werden. Zwischen den Ballons ist ein den Harnleiter verschließbarer bzw. eine Engstelle bildender Stein einschließbar. In ausgedehntem Zustand der Ballons wird der Behandlungskatheter aus dem Harnleiter herausgezogen und dabei der Stein entfernt. Dabei kann es jedoch zu Komplikationen kommen, wenn sich der Stein beispielsweise beim Herausziehen zwischen einen Ballon und die Harnleiterinnenwand einklemmt. Durch Änderung der Ausdehnung der Ballons muß der Stein dann erneut richtig in dem von den Ballons begrenzten Bereich erfaßt werden. Beim Herausziehen wird dabei von der Engstelle ab der gesamte Harnleiter einer starken Dehnung unterworfen und dabei besonders die Innenwand durch starken Reibungsdruck beansprucht. Es kann daher zu Rissen und Ablösungen zumindest der Innenwand des Harnleiters kommen.

Weiterhin ist es auch schon aus der DE-A-3 028 089 zur Erweiterung von Engstellen im Arteriensystem bekannt, einen Behandlungskatheter zu benutzen, an dessen Ende ein in seiner Zirkumferenz definitiv ausdehnbarer Ballon angebracht ist. Dieser Ballon wird im Bereich der Engstelle plaziert und über einen Katheter mit Flüssigkeit gefüllt, so daß das die Engstelle verursachende Gewebe in die Wand des Blutgefäßes hineingedrückt wird und dort verbleibt. Hierbei läßt es sich vielfach nicht vermeiden, daß es während der Dilatation zu Einrissen der Intima - der innersten Wand des Gefäßsystems - kommt. Ferner besteht die Gefahr daß die inneren Gefäßschichten zum Teil abgelöst werden und den freien Innenraum des Blutgefäßes belegen wodurch im Gefäßsystem Verschlüsse und/oder Minderdurchblutungen der Organe entstehen können, die durch das betroffene Blutgefäß versorgt werden. So kann beispielsweise an den Herzkranzgefäßen infolge zu geringer Blutversorgung ein Infarkt entstehen.

Die der vorliegenden Erfindung zugrundeliegende Aufgabe besteht in der Schaffung eines im Aufbau einfachen, wirtschaftlich herstellbaren und zuverlässigen Behandlungskatheters, mit dessen Hilfe die Engstellen beliebiger Körperflüssigkeit führender Gefäße unabhängig von ihrer räumlichen Anordnung oder Ausdehnung im Gefäßsystem so verkleinert bzw. beseitigt werden, daß es zu keinen Einbrüchen oder Rissen in der Gefäßwandung kommen kann. Bei Anwendung des erfindungsgemäßen Behandlungskatheters in Blutgefäßen soll die Versorgung des bzw. der der Engstelle nachgeordneten Organe auch während einer länger andauernden Engstellenbehandlung sichergestellt und damit auch in solchen Fällen eine Infarktgefahr ausgeschlossen werden.

Diese Aufgabe wird erfindungsgemäß durch die gegenüber FR-A-2 350 849 neuen Merkmale des Patentanspruches 1 gelöst.

Durch die Anordnung einer Öffnung zwischen den beiden Ballons und deren Anschluß an einen besonderen, von außen zugänglichen Kanal kann jede Engstelle durch gezielte Auflösung oder Zerkleinerung von Engstellenmaterial und einen oder mehrere anschließende Spülvorgänge der Engstelle in relativ kurzer Zeit schonend beseitigt werden. Hierbei tritt praktisch keine nachteilige Nebenwirkung infolge der Gefäßdehnung auf, da diese im gesunden Bereich des Gefäßes und nur an zwei schmalen Stellen vorgenommen wird. Der erfindungsgemäße Behandlungskatheter kann daher in all denjenigen Fällen angewendet werden, bei denen die Engstelle durch auflösbare und/oder zerkleinerbare Substanzen gebildet ist.

Durch die Erweichung des Engstellenmaterials ist die Abführung desselben aus dem Bereich der Engstelle besonders unproblematisch, da die Gefahr von Beschädigungen der Gefäßwände in diesem Falle auf Grund der Konsistenz des Engstellenmaterials völlig ausgeschlossen ist.

Gemäß einer vorteilhaften Ausgestaltung der Erfindung kann zusätzlich ein Versorgungskanal vorgesehen sein, durch den während der Engstellenbehandlung gleichzeitig eine ausreichende Versorgung des nachgeordneten Organs sichergestellt werden kann. Besonders vorteilhaft ist es, daß mit der Erfindung innerhalb vorbestimmbarer Bereiche von Körperflüssigkeit führenden Gefäßen in einem abgrenzbaren Bereich Behandlungsflüssigkeiten, gegebenenfalls auch in stärker konzentrierter Form, über weitgehend beliebig wählbare Zeiträume auf das Engstellenmaterial zur Einwirkung gebracht werden können.

Vorteilhafte Ausgestaltungen der Erfindung sind Gegenstand der Unteransprüche.

Nachfolgend wird die Erfindung anhand von Ausführungsbeispielen, die in der Zeichnung in stark vergrößertem Maßstab dargestellt sind, näher erläutert. Es zeigen:

Figur 1 im Längsschnitt ein Blutgefäß mit je einem eingeführten Behandlungskatheter, sowie mit den zu beiden Seiten der Verengungsstelle angeordneten Absperrballons,

Figur 2 einen Querschnitt gemäß der Schnittlinie II-II der Figur 1,

Figur 3 einen Längsabschnitt des Behandlungskatheters mit einem darauf verschiebbar angeordneten Ballon,

Figur 4 einen Querschnitt gemäß der Schnittlinie IV-IV der Figur 3,

Figur 5 eine Ansicht eines Behandlungskatheters mit weiteren Trennwänden zur Bildung von zusätzlichen Kanälen für die Zu- und Abführung von Flüssigkeiten oder Gasen,

Figur 6 einen Längsschnitt eines Behandlungskatheterteiles mit teilweise perforierten Mantelflächen bzw. Sollbruchstellen, gemäß der Schnittlinie VI-VI der Figur 5,

Figur 7 einen Längsschnitt durch einen Gefäßabschnitt mit eingeführtem Behandlungskatheter und einem auf diesem verschiebbaren Bereichsbegrenzungskatheter,

Figur 8 einen Querschnitt gemäß der Schnittlinie X-X der Figur 7 und

Figur 9 einen Querschnitt entsprechend einer Schnittlinie X-X der Figur 7, jedoch mit einem Behandlungskatheter mit Einkerbungen im Bereichsbegrenzungskatheter.

Der Behandlungskatheter 1 wird durch einen Führungskatheter 2 in ein Blutgefäß 3 eingeführt. Er überragt den Führungskatheter 2 so weit, daß die auf dem Behandlungskatheter 1 angeordneten Ballons 4 und 5 mit der zwischen ihnen eingeschlossenen Engstelle 6 oder Stenose vor dem Ende des Führungskatheters 2 liegen. Die Engstelle 6 wird üblicherweise aus einem schwammartigen, kalk- und fettreichen Gewebe 7 gebildet, das in der Zeichnung mit Kreuzschraffur angedeutet ist. Für den Fall, daß das Gewebe 7 den Durchgang des Blutgefäßes 3 völlig oder fast völlig verschließt, wird der Behandlungskatheter 1 oder vorab ein anderes Instrument durch dieses hindurchgestoßen. Ansonsten wird der Behandlungskatheter 1 so weit durch die noch vorhandene Öffnung der Engstelle 6 geführt, daß der Ballon 4 im aufgeblasenen Zustand 4' die Engstelle 6 rückseitig und der Ballon 5 im aufgeblasenen Zustand 5' die Engstelle 6 vorderseitig abdichtet, d.h. daß das Blutgefäß 3 zu beiden Seiten der Engstelle 6 ringsherum dicht abgeschlossen ist.

Das Aufweiten bzw. Aufblasen der Ballons 4, 5 kann durch Gas oder ein flüssiges Medium erfolgen. Letzteres kann durch die Ein-/Auslaßdüse S bzw. 9 und über einen durch Längstrennwände 20 gebildeten Druckkanal 10 von außen ein- bzw. ausgelassen werden, wobei deren Menge und Druck regelbar sind. Damit bei der Behandlung von Engstellen 6 in Blutgefäßen trotz der beiderseitigen Verschlüsse der Engstelle 6 die Versorgung des bzw. der nachgeordneten Organe, insbesondere bei längeren Eingriffzeiten sichergestellt ist, ist ein zweiter, als Versorgungskanal 11 ausgebildeter Kanal vorgesehen, der eine Art "Bypass-Leitung" zu dem abgedichteten Engstellenbereich darstellt. Dieser Versorgungskanal 11 ist vorzugsweise koaxial zum Behandlungskatheter 1 angeordnet. Er kann jedoch auch hiervon abweichend in anderer Lage und/oder in Form einer größeren Anzahl von Kanälen ein- oder angebaut werden. Über diesen Versorgungskanal 11 kann Blut und/oder eine andere Flüssigkeit, vorzugsweise bei Überdruck, von außen bzw. mit Hilfe eines Überlaufkanals aus der Blutbahn vor der Blockade einströmen. Hierdurch stehen ausreichend lange Eingriffszeiten zur Verfügung, ohne daß dabei eine Infarktgefahr besteht.

Damit ausschließlich das kalk- und fettreiche Gewebe 7 der Engstelle 6, nicht jedoch die Innenhäute des Blutgefäßes 3 angegriffen werden, wird durch den als Zulaufkanal 12 ausgebildeten Kanal mit der Einlaßöffnung A ein chemisches Mittel in Form einer Lösung in den das Engstellengewebe 7 enthaltenden Raum gepumpt, das in der Lage ist, das Gewebe 7 aufzulösen. Hierzu eignen sich u.a. Verdauungsfermente oder ähnliche Substanzen.

Für andere Anwendungen, wie die Behandlung von Gallen- oder Nierenkonkrementen, kommen auch andere Substanzen, wie lösende Säuren, in Betracht.

Für den Abtransport der gelösten bzw. zerkleinerten Partikel und zur Verkürzung der Behandlungszeit ist eine kontinuierliche Umlaufspülung vorteilhaft, die über einen als Rücklaufkanal 13 ausgebildeten Kanal mit dem Auslaßöffnung B gewährleistet ist. Grundsätzlich ist es aber auch möglich, das das Engstellenmaterial 7 auflösende oder in kleinere Partikel zerteilende Mittel über einen einzigen Kanal zuzuführen und alternierend über denselben Kanal das gelöste oder zerkleinerte Engstellenmaterial 7 abzuführen. Selbstverständlich kann der Zulaufkanal 12 auch als Rücklaufkanal und der Rücklaufkanal 13 auch als Zulaufkanal verwendet werden.

Je nach Bedarf kann die Zahl der Zu- und Rücklaufkanäle auch erhöht werden.

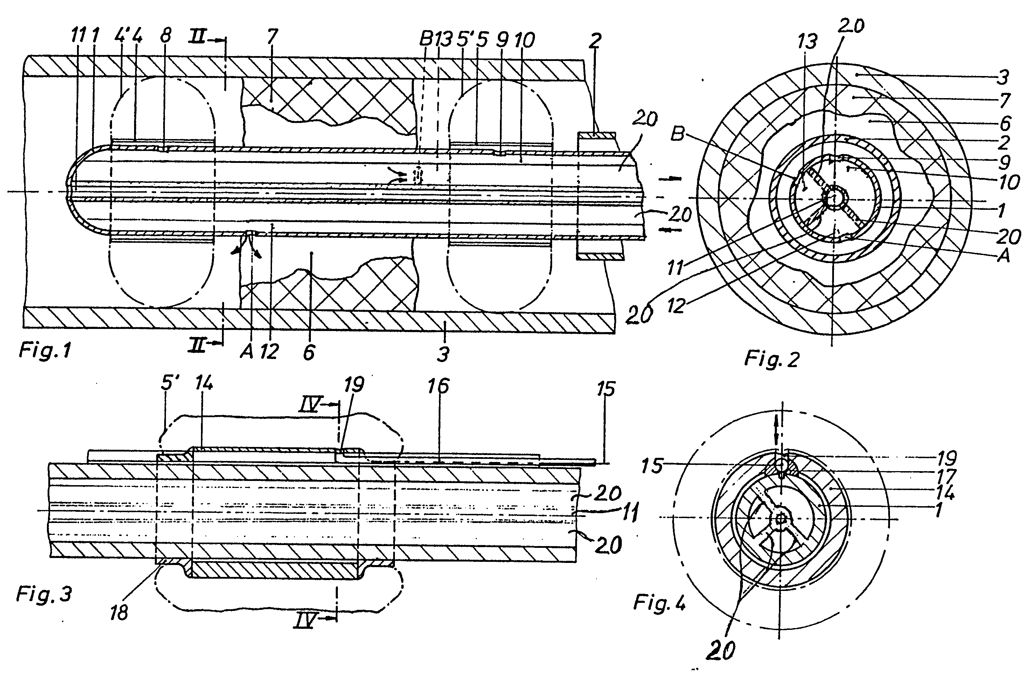

Um die Größe der Blockadestelle der Ausdehnung des Gewebes 7 anpassen zu können, ist es von Vorteil, wenn der Ballon 5 nicht starr- wie in Figur 1 angedeutet - sondern auf dem Behandlungskatheter 1 verschiebbar angeordnet ist. Eine solche Ausführungsform ist in den Figuren 3 und 4 dargestellt. Der Ballon 5 ist dabei auf einem Schieber 14 befestigt, der vorzugsweise mit Hilfe eines steiferen Kunstfaser-, vorzugsweise Perlondrahtes 15, o.dgl. von außen hin- und herbewegbar ist. Der Kunstfaserdraht 15 kann dabei in einem Kanal 16 geführt werden, der beispielsweise in einer gleichzeitig zur Führung des Schiebers 14 dienenden, längsgestreckten Erhebung 17 des Behandlungskatheters 1, also praktisch in seiner Wandverstärkung eingebracht ist. Durch diesen Kanal 16 kann gleichzeitig das Aufblasmedium zu- und abgeführt und dessen Druck von außen geregelt werden.

Damit der Spalt zwischen Schieber 14 und der Außenwand des Behandlungskatheters 1 einwandfrei abgedichtet wird, ist wenigstens an der der Engstelle 6 zugewandten Seite eine Dichtungsmuffe 18 vorgesehen, die durch besondere Formgebung des Ballons 5 zusätzlich zur wirksamen Abdichtung an dieser Dichtungsstelle beiträgt. Mit 19 ist die Düse bezeichnet, über die das gasförmige oder flüssige Medium zum Aufweiten des nunmehr verschiebbaren Ballons 5 zu-bzw. abgeführt wird.

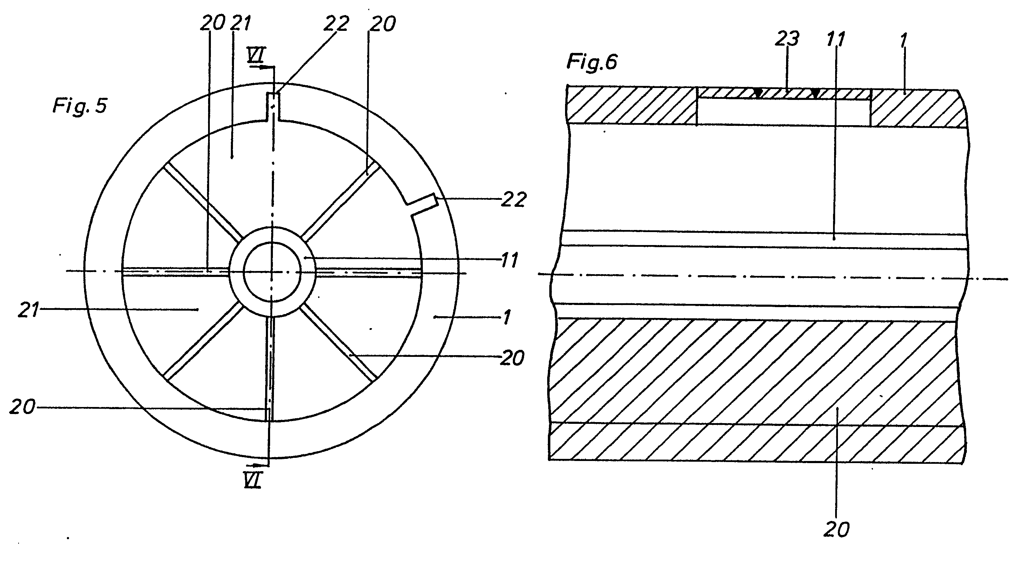

Wie ersichtlich, kann also der Innenraum des Behandlungskatheters 1 durch die Längstrennwände 20 entsprechend unterteilt werden, und wie in Figur 5 gezeigt, können zusätzliche Längstrennwände 20 vorgesehen sein, so daß Kanäle 21 entstehen, durch die, je nach Bedarf, das entsprechende Medium geleitet werden kann.

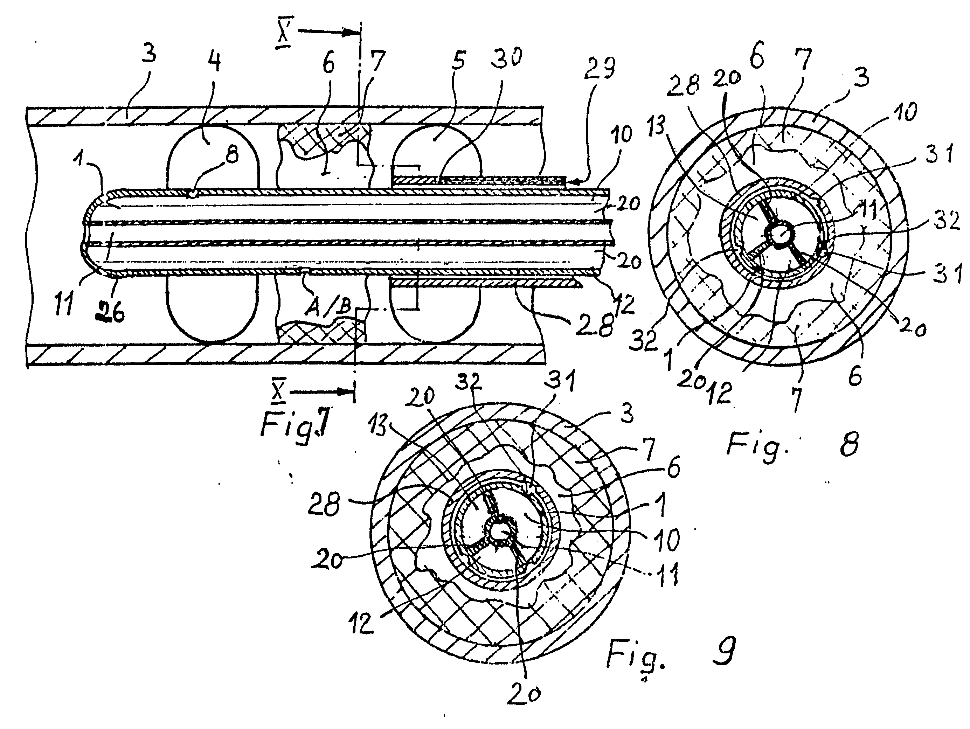

Ferner kann es in manchen Fällen vorteilhaft sein, statt der Anbringung von Ein- Auslaßdüsen S und 9 bzw. der Öffnungen A und B an den Enden der Kanäle 12 und 13, an diesen Stellen die Wandung des Behandlungskatheters 1 mit Perforationen 23 zu versehen, wobei diese, wie aus den Figuren 5 und 6 ersichtlich, erst nach dem Aufbrechen von Sollbruchstellen 22 entstehen, so daß die Länge des perforierten Katheterteiles erst vor dem Eingriff entsprechend der Größe der zu behandelnden Engstelle eingestellt werden kann.

Gemäß einer weiteren vorteilhaften Weiterbildung der Erfindung kann der eine Ballon 4 oder 5 eine elastischere Hülle oder Wand aufweisen als der andere. Dadurch ist es möglich, den Druck so einzustellen und einzuregulieren, daß beide Ballons 4 und 5 abdichten und z.B. beim Abziehen der Substanzen aus dem begrenzten Bereich der Ballon 4 noch abdichtet, der Ballon 5 jedoch nicht mehr. Hierdurch ist es möglich, den Führungskatheter 2 zusätzlich zur Spülung oder Absaugung zu verwenden, da bei einer solchen Druckeinstellung die Engstelle 6 auch von der Seite des Führungskatheters 2 aus zugänglich ist.

Zweckmäßig können die Ballons 4, 5 des erfindungsgemäßen Behandlungskatheters so ausgeführt sein, daß sie eine bestimmte Form und Größe annehmen und dann selbst bei Anwendung eines erheblichen höheren Druckes nicht mehr größer werden.

Bei den in den Figuren 7, 8 und 9 dargestellten vorteilhaften Ausgestaltungen des Erfindungsgegenstandes ist der vom gefäßseitigen Ende 26 des Behandlungskatheters 1 weiter entfernte Ballon 5 am bzw. auf dem Ende eines Bereichsbegrenzungskatheters 28 vorgesehen, der auf dem Behandlungskatheter 1 verschiebbar angeordnet ist. Der Bereichsbegrenzungskatheter 28 ist mit einem Druckkanal 29 versehen, der vorzugsweise in der Wand des Bereichsbegrenzungskatheter 28 integriert ist und der in eine innerhalb des Ballons 5 vorgesehene Öffnung 30 mündet. Durch Verschiebung des Bereichsbegrenzungskatheters 28 kann die Länge des begrenzten Bereiches den Bedürfnissen entsprechend gewählt und durch die getrennte Ausdehnungsmöglichkeit des Ballons 5 abgedichtet oder geöffnet werden. Auch hier kann vorteilhaft ein Raum zwischen dem Behandlungskatheter 1 und dem Begrenzungskatheter 28 belassen und dieser zur Spülung oder Absaugung verwendet werden. Für eine sehr gedrängte Bauweise können hierfür auch eine oder mehrere Einkerbungen 31 an der Innenwand des Bereichsbegrenzungskatheters 28 und/oder Einkerbungen 32 an der Außenwand des Behandlungskatheters 1 vorgesehen sein, wie die Fig. S und 9 zeigen. Um hierbei größere Querschnitte für den Durchfluß zu erzielen, können die Einkerbungen 31 des Bereichsbegrenzungskatheters 28 den Einkerbungen 32 des Behandlungskatheters 1 gegenüber angeordnet sein. Die Behandlungskatheter gemäß den Figuren 7, S und 9 können ferner noch einen nicht dargestellten Führungskatheter 2 (entsprechend Figur 1) aufweisen.

Der erfindungsgemäße Behandlungskatheter für die Behandlung von Engstellen kann bei allen Arten von Körperflüssigkeit führenden Gefäßen, also bei allen Arten von Blutgefäßen oder andere Flüssigkeiten führenden Gefäßen, wie Liquorkanälen oder auch bei größeren Lymphgefäßen eingesetzt werden.

Weitere Anwendungsgebiete - neben dem hauptsächlichen Anwendungsgebiet der Behandlung von Engstellen in Blutgefäßen - sind beispielsweise die Behandlung von Verengungen im Gallengang, im Pankreas-Ausführungsgang oder im Rückenmarkskanal.

Mit der Erfindung ist es auch möglich, chemische Substanzen in hoch- oder höherkonzentrierter Form an Gefäßbereiche mit dem Ziele heranzubringen, von diesen Gefäßbereichen aus Kapillarsprossungen anzuregen. In Betracht kommen hierbei insbesondere die von Herrn Dr. Josef Wissler, Max-Planck-Institut, Bad Nauheim, beschriebenen Stoffe zur Anregung von Gefäßsprossungen, nämlich Angiotropine, die aus weißen Blutkörperchen isoliert werden.

Die Engstellenbehandlung mit dem erfindungsgemäßen Behandlungskatheter kann in mehreren Stufen, gegebenenfalls unter Verwendung unterschiedlicher Behandlungsmedien und/ oder unterschiedlicher Konzentrationen der Behandlungsmedien, vorgenommen werden.

**Patentansprüche**

1. Behandlungskatheter (1) zur Entfernung bzw. Aufweitung von Engstellen in Körperflüssigkeit führenden Gefäßen mit zwei in axialer Richtung mit Abstand von einander angeordneten ausdehnbare Ballons (4, 5) von denen der erste Ballon im aufgeblasenen Zustand (4') den Bereich der Behandlung rückseitig abdichtet und der zweite Ballon im aufgeblasenen Zustand (5') den Behandlungsbereich vorderseitig abdichtet, wobei der Ausdehnungsgrad der beiden Ballons (4, 5) von außen regulierbar ist, sowie mit mindestens einer Öffnung (A,B; A/B) in der Wandung des Behandlungskatheters (1) zwischen den beiden Ballons (4, 5) und mit zwei oder mehr im Innenraum des Behandlungskatheters (1) vorgesehenen Längstrennwänden (20), durch die der Innenraum des Behandlungskatheters (1) in drei oder mehr Kanäle (21) unterteilt ist, von denen einer das Druckmittel für wenigstens den ersten Ballon (4) führt oder zwei das Druckmittel für die beiden Ballons (4, 5) führen und von denen zwei weitere Kanäle (21) zu der Öffnung oder den Öffnungen (A,B; A/B) führen, so daß über den ersten dieser weiteren Kanäle (21) durch die Öffnung oder Öffnungen (A,B; A/B) von außen ein Mittel in den Behandlungsbereich zuführbar ist, das das Material (7) einer Engstelle (6) auflöst, in kleinere Partikel zerteilt und/oder erweicht und über den zweiten dieser weiteren Kanäle durch die Öffnung oder Öffnungen (A,B; A/B) das im Behandlungsbereich aufgelöste, in kleinere Partikel zerteilte und/oder erweichte Engstellenmaterial nach außen abführbar ist.

2. Katheter nach Anspruch 1, dadurch gekennzeichnet, daß zusätzlich wenigstens ein in den Behandlungskatheter (1) integrierter Versorgungskanal (11) vorgesehen ist, der am gefäßseitigen Ende des Behandlungskatheters (1), also nach den beiden Ballons (4, 5), in das Gefäß (3) mündet und von außen zugänglich ist, so daß während der Engstellenbehandlung Substanzen durch den abgedichteten Bereich transportierbar sind.

3. Katheter nach Anspruch 1 oder 2, dadurch gekennzeichnet, daß beide Ballons (4, 5) mit einem gemeinsamen Kanal (21) verbunden sind.

4. Katheter nach Anspruch 3, dadurch gekennzeichnet, daß beide Ballons (4, 5) unterschiedliche Elastizität aufweisen derart, daß mit ansteigendem Druck erst der eine (4 oder 5) und bei höherem Druck auch der andere Ballon (5 oder 4) die absperrende Ausdehnung erreicht und bei nachlassendem Druck der eine (4 oder 5) noch absperrt und der andere (5 oder 4) eine schon nicht mehr absperrende Ausdehnung einnimmt.

5. Katheter nach einem der Ansprüche 1 bis 4, dadurch gekennzeichnet, daß beide Ballons (4, 5) derart ausgestaltet sind, daß sie nur eine begrenzte, zur Absperrung jedoch ausreichende Ausdehnung ausführen können.

6. Katheter nach einem der Ansprüche 1 bis 5, dadurch gekennzeichnet, daß die Ein- und Auslaßöffnungen (A,B) für das Behandlungsmittel durch in die Wandung des Behandlungskatheters (1) eingebrachte Perforationen (23) gebildet sind.

7. Katheter nach einem der Ansprüche 1 bis 6, dadurch gekennzeichnet, daß der den Behandlungsbereich vorderseitig abdichtende zweite Ballon (5) auf der Außenwand des Behandlungskatheters (1) verschiebbar angeordnet ist, und ein separater Kanal (16) zur Führung des Druckmittels vorgesehen ist.

8. Katheter nach Anspruch 7, dadurch gekennzeichnet, daß der bewegliche Ballon (5) auf einem Schieber (14) befestigt ist, der von außen hin- und herbewegbar ist.

9. Katheter nach Anspruch 7 oder 8, dadurch gekennzeichnet, daß der Schieber (14) auf dem Mantel des Behandlungskatheters (1) geführt ist.

10. Katheter nach einem der Ansprüche 7 bis 9, dadurch gekennzeichnet, daß der Schieber (14) als Träger eines Schlauches (16) for die Weiterleitung des Druckmittels für den beweglichen Ballon (5) dient.

11. Katheter nach einem der Ansprüche 7 bis 10, dadurch gekennzeichnet, daß der Schieber (14) wenigstens auf der der Engstelle (6) zugewandten Seite eine Dichtungsmuffe (18) aufweist, die von einem Druckmittel, insbesondere von dem aufgeblasenen Ballon (5'), fest auf die Mantelfläche des Behandlungskatheters (1) gepreßt wird.

12. Katheter nach einem der Ansprüche 7 bis 11, dadurch gekennzeichnet, daß der Schieber als den Behandlungskatheter (1) umschließender, als Bereichsbegrenzungskatheter (28) dienender, weiterer Katheter ausgebildet ist.

13. Katheter nach Anspruch 12, dadurch gekennzeichnet, daß in die Wandung des Bereichsbegrenzungskatheters (28) ein nach außen führender Kanal (29) integriert ist.

14. Katheter nach Anspruch 12 oder 13, dadurch gekennzeichnet, daß wenigstens ein weiterer Zu- und/oder Ablaufkanal (25) für den Behandlungsbereich durch jeweils wenigstens eine Einkerbung (31, 32) in der Innenwandung des Bereichsbegrenzungskatheters (28) und/oder in der Außenwand des Behandlungskatheters (1) gebildet ist.

15. Katheter nach Anspruch 14, dadurch gekennzeichnet, daß die Einkerbung(en) (32) des Behandlungskatheters (1) gegenüber der bzw. den Einkerbung(en) (31) des Bereichsbegrenzungskatheters (28) vorgesehen ist bzw. sind.

**Claims**

1. A treatment catheter (1) for the removal of or dilatation of occlusions in vessels carrying body fluids, comprising two expandable balloons (4, 5), which are spaced apart in the axial direction and the first of which, in the inflated state (4') seals off the treatment zone at the back while the second balloon in the inflated state (5') seals off the treatment zone at the front, the degree of expansion of the two balloons (4, 5) being externally controllable, and comprising at least one opening (A, B; A/B) in the wall of the treatment catheter (1) between the two balloons (4, 5) and two or more longitudinal partitions (20) which are provided in the interior of the treatment catheter (1) and by means of which the interior of the treatment catheter (1) is divided into three or more ducts (21), one of which carries the pressure medium for at least the first balloon (4) or two of which carry the pressure medium for the two balloons (4, 5) and from which two other ducts (21) lead to the opening or openings (A, B; A/B) so that an agent which dissolves the material (7) of an occlusion (6), breaks it up into small particles and/or softens it is adapted to be supplied from outside via the first of said additional ducts (21) through the opening or openings (A, B; A/B), and the occlusion material which in the treatment zone has been dissolved, broken into smaller particles and/or softened is adapted to be discharged to the exterior via the second of said additional ducts through the opening or openings (A, B; A/B)

2. A catheter according to claim 1, characterised in that at least one supply duct (11) incorporated in the treatment catheter (1) is additionally provided and leads into the vessel (3) at the vessel end of the treatment catheter (1) i.e. after the two balloons (4, 5) and is accessible from outside so that during the treatment of the occlusion substances are transportable through the sealed zone.

3. A catheter according to claim 1 or 2, characterised in that the two balloons (4, 5) are connected to a common duct (21).

4. A catheter according to claim 3, characterised in that the two balloons (4, 5) have different elasticity so that as the pressure rises first one of the two balloons (4 or 5) reaches the sealing expansion and, at higher pressure, the other balloon (5 or 4) also reaches the sealing expansion and as the pressure relaxes one of the balloons (4 or 5) still seals and the other (5 or 4) assumes an expansion which at this stage no longer seals.

5. A catheter according to any one of claims 1 to 4, characterised in that the two balloons (4, 5) are so devised that they can perform only a limited expansion, but one sufficient for sealing.

6. A catheter according to any one of claims 1 to 5, characterised in that the inlet and outlet openings (A, B) for the treatment agent are formed by perforations (23) in the wall of the treatment catheter (1).

7. A catheter according to any one of claims 1 to 6, characterised in that the second balloon (5) which seals the treatment zone at the front is disposed slidably on the outer wall of the treatment catheter (1) and a separate duct (16) is provided to carry the pressure medium.

8. A catheter according to claim 7, characterised in that the movable balloon (5) is fixed on a slide (14) which is adapted to reciprocate from outside.

9. A catheter according to claim 7 or 8, characterised in that the slide (14) is guided on the casing of the treatment catheter (1).

10. A catheter according to any one of claims 7 to 9, characterised in that the slide (14) acts as a carrier for a hose (16) for transmission of the pressure medium for the movable balloon (5).

11. A catheter according to any one of claims 7 to 10, characterised in that the slide (14) has a sealing sleeve (18) at least on the side facing the occlusion (6), said sleeve being pressed by a pressure medium, more particularly the inflated balloon (5,), firmly on to the outer surface of the treatment catheter (1).

12. A catheter according to any one of claims 7 to 11, characterised in that the slide is constructed as another catheter which encloses the treatment catheter (1) and which serves as a zone limiting catheter (28).

13. A catheter according to claim 12, characterised in that a duct (29) leading to the exterior is incorporated in the wall of the zone limiting catheter (28).

14. A catheter according to claim 12 or 13, characterised in that another supply and/or discharge duct (25) for the treatment zone is formed by, in each case, at least one notch (31, 31) in the inner wall of the zone limiting catheter (28) and/or in the outer wall of the treatment catheter (1).

15. A catheter according to claim 14, characterised in that the or each notch (32) of the treatment catheter (1) is provided opposite the or each notch (31) of the zone limiting catheter (28).

**Revendications**

1. Cathéter de traitement (1) pour la suppression ou la dilatation de zones de resserrement dans des vaisseaux porteurs de fluides corporels, comprenant deux ballons dilatables (4, 5) situés à distance l'un de l'autre dans le sens axial, parmi lesquels le premier ballon assure par-derrière en condition gonflée (4') l'étanchéité de la région du traitement, et le second ballon assure par-devant en condition gonflée (5') l'étanchéité de cette région traitée, le degré de dilatation dés deux ballons (4, 5) pouvant être régulé de l'extérieur, ainsi qu'au moins un orifice (A, B; A/B) pratiqué dans la paroi du cathétér de traitement (1) entre les deux ballons (4, 5), ainsi que deux cloisons séparatrices longitudinales (20) ou plus, prévues dans l'espace interne du cathéter de traitement (1) et par l'intermédiaire desquelles cet éspace interne du cathéter de traitement (1) est subdivisé en trois canaux (21) ou plus, dont l'un achemine le fluide sous pression au moins pour le premier ballon (4) ou deux acheminent le fluide sous pression pour les deux ballons (4, 5), et parmi lesquels deux autres canaux (21) gagnent l'orifice ou les orifices (A, B; A/B), de sorte que, par l'intermédiaire du premier de ces autres canaux (21), un agent peut être délivré de l'extérieur dans la région de traitement en franchissant l'orifice ou les orifices (A, B; A/B), cet agent provoquant la dissolution, la fragmentation en de petites particules et/ou le ramollissement de la matière (7) constituant une zone de resserrement (6), tandis que, par l'intermédiaire du second de ces canaux supplémentaires, la matière de la zone de resserrement dissoute, fragmentée en de petites particules et/ou ramollie dans la région du traitement peut, en franchissant l'orifice ou les orifices (A, B; A/B), être évacuée vers l'exterieur.

2. Cathéter selon la revendication 1, caractérisé par le fait qu'il est prévu, en plus, au moins un canal d'irrigation (11) intégré dans le cathéter de traitement (1), qui débouche dans le vaisseau (3) à l'extrémité du cathéter de traitement (1) située côté vaisseau, c'est-à-dire vers les deux ballons (4, 5), et est accessible de l'extérieur, de sorte qué, au cours du traitément de la zone de resserrement, des substances peuvent être acheminées à trarers la région rendue étanche.

3. Cathéter sélon la revendication 1 ou 2, caractérisé par le fait que les deux ballons (4, 5) sont relies a un canal commun (21).

4. Cathéter selon la revendication 3, caractérisé par le fait que les déux ballons (4, 5) présentent dés élasticités différentes de telle sorte que, lorsque la pression croit, tout d'abord l'un (4 ou 5) et, lorsque la pression est supérieure, également l'autre ballon (5 ou 4) atteigne la dilatation d'obstruction, et que, lorsque la pression décroit, l'un (4 ou 5) continue d'assurer l'obstruction et l'autre (5 ou 4) prenne une condition de dilatation qui n'assure déjà plus l'obstruction.

5. Cathéter selon l'une des revendications 1 à 4, caractérisé par le fait que les deux ballons (4, 5) sont réalisés de manière qu'ils ne puissent atteindre qu'une dilatation limitée, qui est neanmoins suffisante pour provoquer l'obstruction.

6. Cathéter selon l'une des revendications 1 à 5, caractérisé par le fait que les orifices (A, B) d'admission et de sortie de l'agent de traitement sont formés par des perforations (23) pratiquées dans la paroi du cathéter de traitement (1).

7. Cathéter selon l'une des revendications 1 à 6, caractérisé par le fait que le second ballon (5) assurant par-devant l'étanchéité de la région du traitement est disposé avec faculté de coulissement sur la paroi externe du cathéter de traitement (1), un canal séparé (16) étant prévu pour guider le fluide sous pression.

8. Cathéter selon la revendication 7, caractérisé par le fait que le ballon mobile (5) est fixé sur une pièce coulissante (14) à laquelle un mouvement alternatif peut être imprimé de l'extérieur.

9. Cathéter selon la revendication 7 ou 8, caractérisé par le fait que la pièce coulissanté (14) est guidée sur l'enveloppe du cathéter de traitement (1).

10. Cathéter selon l'une des revendications 7 à 9, caractérisé par le fait que la pièce coulissante (14) sert, en tant que support d'un flexible (16), au transfert du fluide sous pression destiné au ballon mobilé (5).

11. Cathéter selon l'une des revendications 7 à 10, caractérisé par le fait que la pièce coulissante (14) présente, au moins du côté tourné vers la zone de resserrement (6), un manchon d'étanchement (18) qui est pressé fermement, sur la surface de l'enveloppe du cathéter de traitement (1), par un fluide sous pression et notamment par le ballon gonflé (5').

12. Cathéter selon l'une des revendications 7 à 11, caractérisé par le fait que la pièce coulissante est réalisée sous la forme d'un autre cathéter qui éntoure le cathéter de traitement (1) et sert de cathéter (28) de délimitation de zones.

13. Cathéter selon la revendication 12, caractérisé par le fait qu'un canal (29) gagnant l'extérieur est intégré dans la paroi du cathéter (28) de délimitation de zones.

14. Cathéter selon la revendication 12 ou 13, caractérisé par le fait qu'au moins un autre canal (25) de délivrance et/ou d'évacuation pour la région du traitement est formé, à chaque fois, par au moins une encoche (31, 32) pratiquée dans la paroi interne du cathéter (28) de délimitation de zones et/ou dans la paroi externe du cathéter de traitement (1).

15. Cathéter selon la revendication 14, caractérisé par le fait que la ou les encoches (32) du cathéter de traitement (1) est ou sont prévues en regard de la ou des encoches (31) du cathéter (28) de délimitation de zones.

11 1 4'4 8 II 7 B13 5'5 9 10 2

20

20

20

Fig. 1

II A 12 6 3

0 080 436

13 20

B

3
7
6
2
9
10

1
20
A

11
20 12

Fig. 2

5' 14 IV 19 16 15

20
11
20

Fig. 3

18 IV

19
17
14
1

15

20

Fig. 4

Fig. 5

Fig. 6

0 080 436

Fig. 7

Fig. 8

Fig. 9

0 080 436